# FASCICULE DE BREVET EUROPEEN

(11) **EP 2 146 662 B1**
(45) Date de publication et mention de la délivrance du brevet: **24.10.2018**
(21) Numéro de dépôt: 08805728.6
(22) Date de dépôt: 30.04.2008
(51) Int. Cl.: A61B 17/16, A61C 3/03, A61C 8/00, A61C 8/02, A61B 17/3203

(54) **INSERT ULTRASONIQUE DE SOULÈVEMENT DE MEMBRANE SINUSIENNE**
ULTRASCHALLEINSATZ ZUR ANHEBUNG EINER SINUS-MEMBRAN
ULTRASOUND INSERT FOR LIFTING SINUS MEMBRANE

(30) Priorité: 04.05.2007 FR 0754881
(43) Date de publication de la demande: 27.01.2010
(73) Titulaire: SOCIETE POUR LA CONCEPTION DES APPLICATIONS DES TECHNIQUES ELECTRONIQUES, 33700 Merignac (FR)
(72) Inventeur: KURREK, Andreas, 40878 Ratingen (DE); WAINWRIGHT, Marcel, A., 40489 Dusseldorf (DE); TROEDHAN, Angelo, A-1050 Wien (AT); DIERAS, Francis, F-33000 Bordeaux (FR)
(74) Mandataire: Desormiere, Pierre-Louis
(86) Numéro de dépôt international: PCT/FR2008/050774
(87) Numéro de publication internationale: WO 2008/148979

(56) Documents cités:
- EP-A- 1 464 311
- WO-A-03/045469
- WO-A-2006/096900
- DE-A1-102005 009 802
- DE-U1- 20 019 711
- FR-A- 2 474 304
- US-A1- 2006 235 305

## Description

### Arrière plan de l'invention

La présente invention concerne le domaine des implants dentaires et plus particulièrement les dispositifs utilisés lors de la préparation d'un site d'implantation en odontologie.

Afin d'être correctement intégré dans l'os maxillaire (ostéo-intégration), l'implant doit pouvoir être fixé dans un volume d'os cortical suffisant. Or, l'os maxillaire ne présente pas toujours une épaisseur ou un volume suffisant d'os cortical pour fixer stablement l'implant. Dans ce cas, il faut procéder à un apport de substance osseuse sur l'os maxillaire du côté du sinus. L'apport osseux peut provenir du patient lui-même (greffon osseux autogène) ou d'une source extérieure (greffon osseux artificiel).

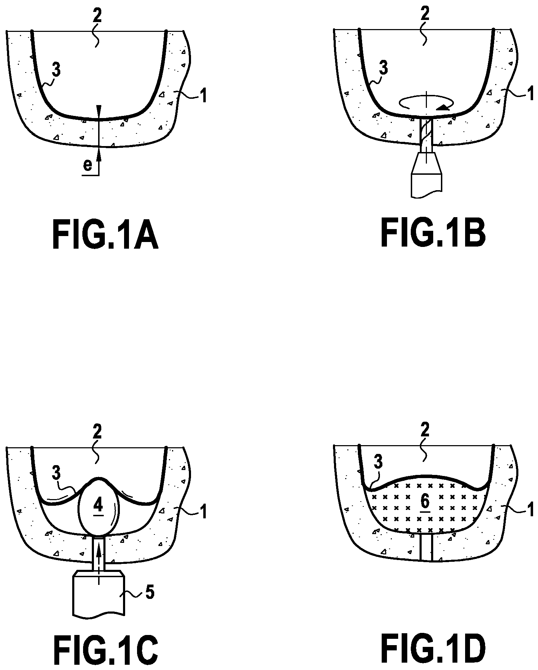

Les figures 1A à 1D montrent les différentes étapes à mettre en oeuvre pour permettre un tel apport osseux dans un os maxillaire d'une mâchoire supérieure d'un patient. La figure 1A représente un os maxillaire 1 qui délimite une cavité correspondant à un sinus 2. Un implant dentaire doit être fixé dans la partie inférieure de l'os maxillaire 1. L'os maxillaire 1 présente à cet endroit une épaisseur e d'os cortical insuffisante pour recevoir l'implant si bien qu'un ajout de substance osseuse du côté du sinus 2 est nécessaire. Après avoir dégagé la gencive (non représentée), le praticien perce tout d'abord l'os maxillaire jusqu'à déboucher dans le sinus 2 (figure 1B). L'os maxillaire 1 est séparé du sinus 2 par le plancher sinusien qui est constitué par une membrane sinusienne 3, encore appelée membrane de Schneider, en contact avec la paroi interne de l'os maxillaire 1. Une fois l'os maxillaire percé, il faut élever la membrane sinusienne 3 de manière à ménager un volume pour l'introduction de la substance osseuse à ajouter. A cet effet, on utilise des instruments permettant d'élever la membrane sinusienne.

Comme illustré sur la figure 1C, l'élévation de la membrane sinusienne 3 peut être réalisée avec ballonnet 4 introduit dans le sinus 2 au moyen d'un instrument 5 via le forage de l'os maxillaire 1. Le gonflement du ballonnet 4 permet de repousser la membrane sinusienne et de dégager un volume entre cette dernière et l'os cortical du maxillaire 1, ce volume étant ensuite comblé avec un greffon osseux 6 comme illustrée sur la figure 1D.

La membrane sinusienne peut être également repoussée avec d'autres types d'instruments notamment de forme contondante.

L'élévation de la membrane sinusienne est une opération très délicate car celle-ci est très fragile et peut à tout moment se déchirer sous l'effet de l'instrument utilisé avec lequel elle est directement en contact. Un tel déchirement compromet sérieusement la réussite de l'implantation notamment en raison de la dispersion de la substance osseuse de substitution dans le sinus et des risques d'infection encourus.

Les instruments connus actuellement pour élever la membrane, même ceux utilisant un ballonnet, sont en contact avec seulement une surface réduite de la membrane si bien que cette dernière peut être facilement déchirée lorsque le praticien exerce une pression trop forte avec l'instrument. Le contrôle de la pression exercée sur la membrane par le praticien est d'autant plus difficile en raison du manque de vision du site et de la profondeur de sa voie d'accès. Certains instruments peuvent en outre être trop invasifs.

Le document DE10 2005 009 802 décrit un appareil de traitement dentaire à ultrasons comprenant au moins une pièce à main chirurgicale reliée à un générateur d'ultrasons comportant des moyens pour alimenter la pièce à main en liquide d'irrigation, ladite pièce à main comprenant des moyens pour générer des vibrations ultrasonores et des moyens pour distribuer le liquide d'irrigation délivré par le générateur d'ultrasons. Cet appareil comprend en outre au moins un insert ultrasonique destiné à soulever une membrane sinusienne par introduction dans un trou formé dans un os maxillaire, ledit insert comprenant un corps s'étendant entre une partie proximale adaptée pour être couplée mécaniquement avec une pièce à main chirurgicale génératrice de vibrations ultrasonores et une partie distale destinée à reproduire les vibrations ultrasonores transmises par la pièce à main. La partie distale de l'insert comporte à son extrémité un disque plat en saillie par rapport au reste de l'insert. L'insert comprend de plus un canal d'irrigation interne débouchant au centre de ce disque plat, le canal d'irrigation interne étant en communication avec les moyens de distribution de liquide d'irrigation de ladite pièce à main et ledit insert étant couplé mécaniquement aux moyens de génération de vibrations ultrasonores de la pièce.

### Objet et résumé de l'invention

La présente invention a pour but de proposer une nouvelle conception d'instrument qui permet d'élever la membrane sinusienne sans risque de déchirure de celle-ci.

Ce but est atteint grâce à un insert ultrasonique comprenant un corps s'étendant entre une partie proximale adaptée pour être couplée mécaniquement avec une pièce à main chirurgicale génératrice de vibrations ultrasonores et une partie distale destinée à reproduire les vibrations ultrasonores transmises par la pièce à main, caractérisé en ce que la partie distale comporte une portion annulaire de section croissante dont l'extrémité est formée par une surface plane sensiblement perpendiculaire à l'axe longitudinal de ladite partie distale et en ce que le corps présente une courbure entre la partie proximale et la partie distale, l'insert comprenant en outre un canal d'irrigation interne débouchant sur ladite surface plane.

Ainsi, l'insert ultrasonique de la présente invention présente une conception qui permet une élévation de la membrane sinusienne en toute sécurité. En effet, la partie distale de l'instrument qui est destinée à être introduite sous la membrane sinusienne pour son élévation présente une surface plane de sorte que, même en cas de contact entre la partie distale de l'insert et la membrane, les risques de déchirure de cette dernière sont considérablement réduits en comparaison avec les instruments habituellement utilisés qui présente au niveau de leur portion de contact avec la membrane une forme contondante ou similaire.

En outre, l'insert selon l'invention, comprend un canal d'irrigation interne qui débouche au niveau de la surface plane de la partie distale. Ainsi, lorsque l'insert de l'invention est introduit dans le forage de l'os maxillaire sous la membrane sinusienne, un liquide d'irrigation peut être introduit entre la surface plane de l'insert et la membrane sinusienne qui est alors soulevée par la mise en cavitation du liquide d'irrigation. En effet, lorsque des vibrations ultrasonores sont transmises à l'insert, le liquide d'irrigation entre en cavitation juste en dessous de la membrane sinusienne. L'effet de cavitation (micro-pressions et dépressions) provoque un soulèvement progressif et en douceur de la membrane. Par conséquent, l'insert ultrasonique de l'invention permet de réaliser une élévation de la membrane sinusienne sans contact direct avec celui-ci, ce qui minimise encore les risques de déchirure de la membrane. La forme plate de l'extrémité de la section annulaire permet d'optimiser l'efficacité de l'effet des ultrasons sur le liquide d'irrigation.

Par ailleurs, l'insert présente une courbure au voisinage de sa partie centrale, c'est-à-dire entre la partie proximale et la partie distale de l'insert. Cette courbure permet non seulement de faciliter l'introduction de l'insert dans la cavité de l'os maxillaire mais aussi d'assurer une bonne élévation de la membrane sinusienne. En effet, pour atteindre la membrane sinusienne et procéder à son élévation, l'insert de l'invention doit être introduit dans l'os de la mâchoire supérieure à partir de la bouche du patient. Grâce à la courbure présentée par le corps de l'insert, l'extrémité libre de l'insert comportant la portion annulaire peut être facilement introduite dans le forage ménagé dans l'os maxillaire sans avoir à introduire dans la bouche du patient le reste de l'insert (partie distale) ou une partie de la pièce à main, ce qui améliore grandement le confort pour le patient. En outre, grâce à cette courbure, le praticien peut maintenir la surface plane de l'extrémité libre de l'insert dans une position sensiblement parallèle à la membrane sinusienne à élever, ce qui est important pour obtenir une élévation uniforme de la membrane. Avec un insert présentant un corps droit, le praticien serait gêné par la mâchoire inférieure du patient et aurait des difficultés à introduire l'insert dans le forage de la mâchoire supérieure tout en maintenant la surface plane à l'extrémité de ce dernier dans une position sensiblement parallèle à la membrane sinusienne. L'angle de courbure formé par le corps de l'insert entre la partie proximale et la partie distale est d'au moins 45°.

Selon un aspect particulier de l'invention, le canal d'irrigation interne débouche au centre de la surface plane, ce qui permet de créer une cavitation homogène du liquide d'irrigation.

Selon un autre aspect de l'invention, la surface plane présente à sa périphérie un bord arrondi de manière à minimiser encore les risques de déchirure de la membrane en cas de contact avec cette partie de l'insert.

La surface plane à l'extrémité de la portion annulaire présente un diamètre qui est de préférence proche, c'est-à-dire légèrement inférieur, de celui du forage réalisé dans l'os pour permettre l'introduction de ladite portion annulaire. La surface plane peut, par exemple, présenter un diamètre de 3 mm environ.

L'invention a également pour objet un appareil de traitement dentaire à ultrasons comprenant au moins une pièce à main chirurgicale reliée à un générateur d'ultrasons comportant des moyens pour alimenter la pièce à main en liquide d'irrigation, ladite pièce à main comprenant des moyens pour générer des vibrations ultrasonores et des moyens pour distribuer le liquide d'irrigation délivré par le générateur d'ultrasons, caractérisé en ce qu'il comprend en outre au moins un insert ultrasonique tel que décrit précédemment, ledit insert étant couplé mécaniquement aux moyens de génération de vibrations ultrasonores de la pièce à main, le canal d'irrigation interne de l'insert étant en communication avec les moyens de distribution de liquide d'irrigation de ladite pièce à main.

L'invention concerne également un procédé d'élévation d'une membrane sinusienne disposée entre une cavité sinusale et un os maxillaire, ledit procédé comprenant la réalisation dans l'os maxillaire d'un forage débouchant sous ladite membrane sinusienne caractérisé en ce qu'il comprend en outre des étapes consistant à introduire dans ledit forage un insert ultrasonique selon l'invention, à transmettre des vibrations ultrasonores audit l'insert et à alimenter le canal d'irrigation interne dudit insert avec un liquide d'irrigation.

Ainsi, avec le procédé de l'invention, il est possible de réaliser une élévation uniforme de la membrane sinusienne, et ce en toute sécurité. En effet, une fois l'insert introduit dans le forage de l'os maxillaire sous la membrane sinusienne, le liquide d'irrigation débouchant au niveau de la surface plane de l'insert entre en cavitation sous l'effet des vibrations ultrasonores. Cet effet de cavitation (micro-pressions et dépressions) provoque un soulèvement uniforme et progressif de la membrane sinusienne sans qu'il soit nécessaire d'avoir un contact entre l'insert et la membrane.

### Brève description des dessins

D'autres caractéristiques et avantages de l'invention ressortiront de la description suivante de modes particuliers de réalisation de l'invention, donnés à titre d'exemples non limitatifs, en référence aux dessins annexés, sur lesquels :
- les figures 1A à 1D, déjà décrites, montrent une opération d'ajout de matière osseuse dans un os maxillaire selon l'art antérieur,
- les figures 2 et 3 sont respectivement des vues en coupe et en perspective d'un insert ultrasonique conformément à un mode de réalisation de l'invention,
- la figure 4 représente un appareil de traitement à ultrason équipé de l'insert des figures 2 et 3,
- la figure 5 est une vue en coupe de la pièce à main de la figure 4, et
- les figures 6A à 6D montrent une opération d'ajout de matière osseuse dans un os maxillaire dans laquelle l'élévation d'une membrane sinusienne est réalisée avec un insert selon l'invention.

### Description détaillée des modes de réalisation de l'invention

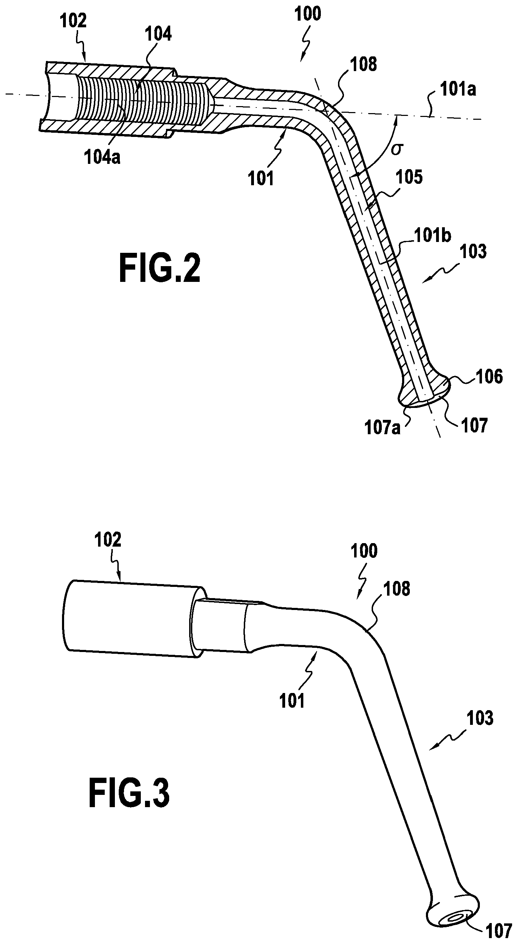

Les figures 2 et 3 représentent un insert ultrasonique 100 pour soulèvement de membrane sinusienne conformément à un mode de réalisation de la présente invention.

L'insert ultrasonique 100 est formé d'un corps 101, par exemple en matériau métallique, qui s'étend entre une partie proximale 102 destinée à être couplée mécaniquement avec une pièce à main chirurgicale génératrice de vibrations comme expliqué plus loin et une partie distale 103 destinée à reproduire les vibrations transmises par la pièce à main. La partie proximale 102 qui s'étend suivant un axe longitudinal 101a comporte une cavité 104 sur la paroi de laquelle est formée un filetage 104a permettant de fixer solidairement l'insert sur la pièce à main. La cavité 104 coopère avec un canal d'irrigation interne 105 qui s'étend à l'intérieur du corps 101 sur toute sa longueur et débouche au niveau de l'extrémité de la partie distale 103.

La partie distale 103 de l'insert 100 qui s'étend suivant un axe longitudinal 101b correspond à la partie de l'insert qui est introduite dans la bouche du patient pour atteindre le forage réalisé dans l'os et procéder à l'élévation de la membrane sinusienne. Dans le mode de réalisation présenté dans les figures 2 et 3, la partie distale 103 comporte une portion annulaire 106 de révolution autour de l'axe 101b qui est, à cet endroit de l'insert, confondu avec l'axe du canal d'irrigation interne 105. La portion annulaire 106 présente une section qui s'agrandit vers l'extrémité libre de partie distale 103. L'extrémité de la portion annulaire 106 de section croissante présente une surface plane 107 qui correspond à la partie de l'insert qui sera placée en regard de la membrane sinusienne à soulever. La surface plane 107 présente de préférence à sa périphérie un bord arrondi 107a plutôt qu'un bord saillant, ce qui permet de limiter les risques d'endommagent sur la membrane sinusienne en cas de contact entre l'insert et cette dernière. Par ailleurs, l'insert présente au moins au niveau de la portion annulaire une surface externe lisse, c'est-à-dire qui n'est pas structurée ni recouverte d'un matériau abrasif afin d'éviter tout risque d'endommagement de la membrane.

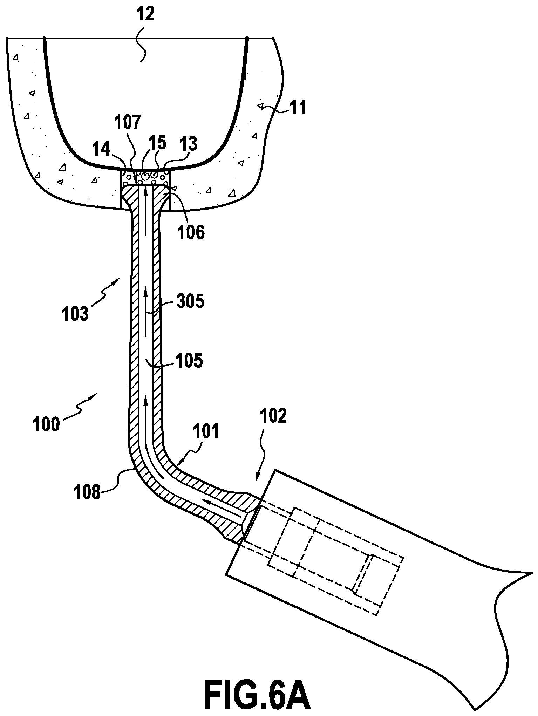

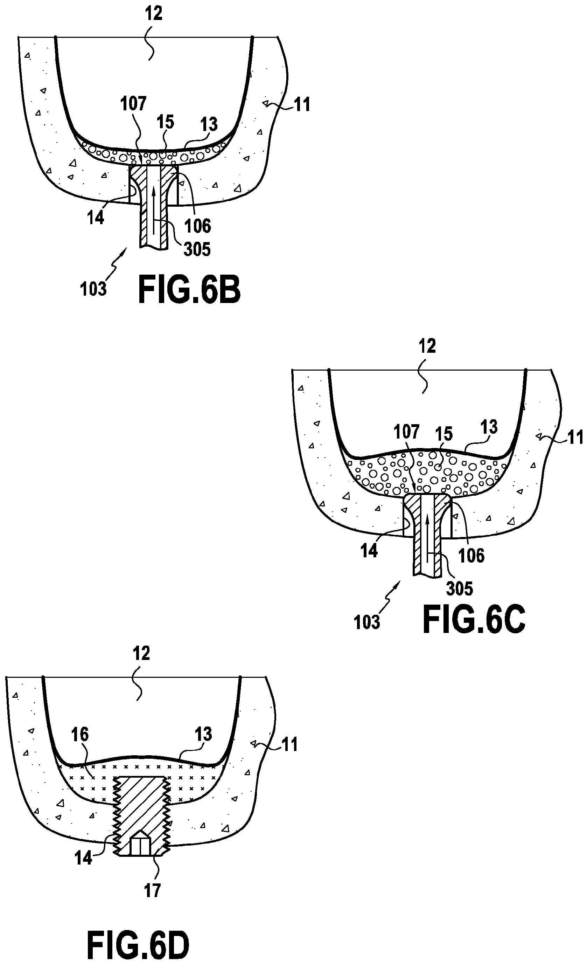

Comme représentée sur la figure 2, le corps 100 présente une portion courbée 108 entre la partie proximale 102 et la partie distale 101. Plus précisément, l'axe longitudinal 101b de la partie distale 103 forme avec l'axe longitudinal 101a de la partie proximale 102 un angle de courbure σ d'au moins 45°. Une telle inclinaison de la partie distale 103 de l'insert 100 permet au praticien à la fois d'introduire facilement l'insert dans le forage ménagé dans l'os maxillaire à partir de la bouche du patient et d'assurer le maintien de la surface plane 107 dans une position sensiblement parallèle à la membrane sinusienne telle qu'illustrée sur les figures 6A et 6B décrites plus loin.

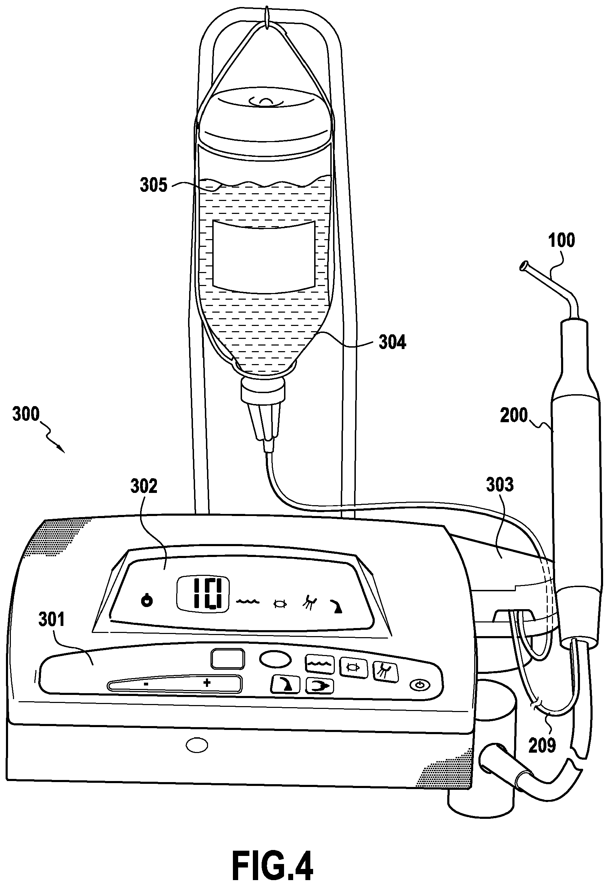

La figure 4 illustre un appareil de traitement à ultrasons qui comprend un générateur d'ultrasons 300 relié à une pièce à main 200 équipé de l'insert ultrasonique 100 décrit précédemment.

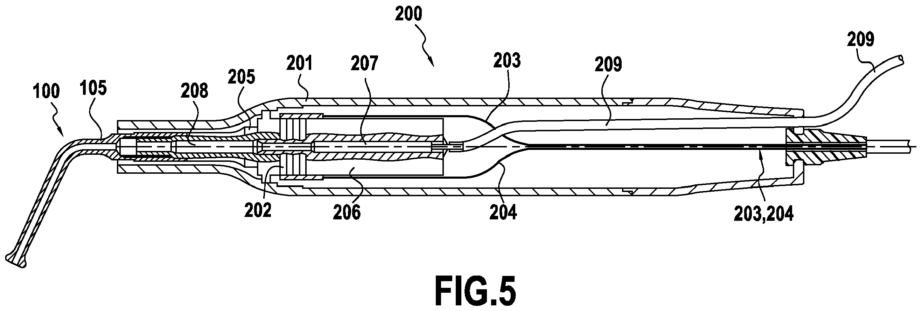

Comme représentée sur la figure 5, la pièce à main 200 est constituée d'un corps creux cylindrique 201 en un matériau isolant qui renferme, de façon connue, un transducteur 202, par exemple constitué de pastilles piézoélectriques, qui est relié électriquement au générateur d'ultrasons 300 par des conducteurs d'alimentation électrique 203, 204.

Un amplificateur de vibration 205 est en contact avec la face antérieure du transducteur 202. L'insert 100 est fixé, par exemple par vissage, à l'extrémité de l'amplificateur 205 de manière à être couplé mécaniquement avec le transducteur 202. L'insert 100 est alors soumis à un mouvement vibratoire longitudinal lorsque le transducteur 202 est alimenté avec un courant alternatif de fréquence élevée fourni et contrôlé par le générateur 300. La puissance et l'amplitude des ondes ultrasonores transmises à l'insert sont réglées à partir du générateur 300 au moyen d'organes de commande 301 (boutons) et d'un dispositif d'affichage 302 des commandes sélectionnées.

Le transducteur 202 est en contact avec une contre-masse 206 au centre de laquelle est ménagé un canal 207 qui communique, d'un côté, avec un canal 208 ménagé dans l'amplificateur 205 et, de l'autre côté avec un tuyau souple 209 relié à une pompe 303 du générateur d'ultrasons 300. Le générateur d'ultrasons 300 comprend en outre une source 304 d'un liquide d'irrigation 305 qui est reliée à la pompe 303. Par conséquent, lorsque la pompe 303 est activée (à partir des organes de commande 301) le liquide d'irrigation 305 de la source 304 est envoyé successivement dans le tuyau 209, le canal 207 de la contre-masse et le canal 208 de l'amplificateur 205 qui communique avec le canal d'irrigation interne 105 de l'insert 100.

Le fonctionnement d'un tel appareil de traitement à ultrasons est bien connu et ne sera pas décrit plus en détail par souci de simplification.

On décrit maintenant en référence aux figures 6A à 6D, une opération d'élévation d'une membrane sinusienne réalisée avec l'insert ultrasonique et l'appareil de traitement à ultrasons décrits précédemment. Lors de cette opération, le praticien utilise un appareil de traitement à ultrasons similaire à celui présenté sur les figures 4 et 5, les positionnements et les déplacements de l'insert illustrés sur les figures 6A à 6C sont réalisés manuellement par le praticien qui tient le dispositif au niveau de la pièce à main.

La figure 6A montre le début de l'opération d'élévation d'une membrane sinusienne 13 séparant un sinus 12 d'un os maxillaire 11. La portion annulaire 106 à section croissante de la partie distale 103 de l'insert 100 est introduite dans le forage 14 réalisé préalablement dans l'os maxillaire 11 pour permettre d'atteindre la membrane sinusienne 13. Une fois la portion annulaire 106 introduite dans le forage 14, le praticien commande le générateur d'ultrasons à la fois pour mettre l'insert en vibrations ultrasonores et pour alimenter le site en liquide d'irrigation via le canal d'irrigation interne 105 de l'insert. La surface plane 107 au centre de laquelle débouche le canal d'irrigation interne 105 permet, lorsque des vibrations ultrasonores sont transmises à l'insert et qu'un liquide d'irrigation débouche du canal 105, de créer une cavitation juste en dessous de la membrane sinusienne et de provoquer son élévation sans contact avec l'insert. En effet, lorsque le liquide d'irrigation 305 débouche à la sortie du canal 105, il se trouve entre la membrane sinusienne 13 et la surface plane 107, cette dernière étant animé d'un mouvement vibratoire ultrasonore. De façon connue, l'action des ultrasons dans les milieux liquides repose sur le phénomène de cavitation qui permet la création, la croissance et l'implosion de bulles formées lorsqu'un liquide est soumis à une onde de pression périodique. Sous l'effet des vibrations ultrasonores de la surface plane 107, le liquide d'irrigation 305 forme des bulles de cavitation hydrodynamique 15 (micro-pressions) qui implosent (dépressions) au contact des surfaces solides rencontrées et notamment au contact de la membrane sinusienne 13. Ces micro-pressions et ces dépressions créent un effet pneumatique sur la membrane qui entraîne un soulèvement progressif et en douceur de cette dernière.

Grâce à la présence de la surface plane 107, il y a peu de risque de déchirer la membrane sinusienne en cas de contact entre l'insert et cette dernière, ce qui n'est pas les cas avec des instruments de forme contondante ou similaire.

En outre, on constate que le soulèvement de la membrane est obtenu par la cavitation du liquide d'irrigation 305 situé entre l'insert et la membrane, c'est-à-dire sans contact entre la membrane sinusienne 13 et l'insert 100, ce qui réduit encore les risques de déchirure de la membrane par l'insert.

Les figures 6B et 6C montrent l'élévation progressive de la membrane sinusienne 13 obtenue notamment en avançant légèrement la surface plane 107 à l'intérieur du sinus 12.

L'utilisation du liquide d'irrigation permet d'obtenir une élévation relativement uniforme sur toute la largeur de la membrane. En effet, comme illustré sur la figure 6B, le liquide d'irrigation 305 introduit sous la membrane 13 se répand dans tout le fond du sinus 12. Le liquide d'irrigation exerce alors une force d'élévation sensiblement uniforme sur une large surface de la membrane, ce qui permet un soulèvement équilibré de celle-ci contrairement au cas où l'on utilise des instruments qui ne permettent d'avoir qu'un point d'appui ponctuel sur une petite partie de la membrane. Par ailleurs, afin d'obtenir une élévation uniforme de la membrane sinusienne au moins dans les premiers instants de l'opération d'élévation, la surface plane 107 de l'insert 100 doit être introduite et maintenue dans une position sensiblement parallèle à la membrane comme représentée sur les figures 6A et 6B. La portion courbée 108 de l'insert 100 permet d'assurer facilement un tel positionnement car l'insert peut être orienté ou positionné par le praticien sans être gêné par la mâchoire inférieure du patient.

Lorsque qu'un volume suffisant a été dégagé sous la membrane, on comble ce volume avec un greffon osseux 16 permettant la pose d'un implant 17 comme illustré sur la figure 6D.

Le corps de l'insert ultrasonique 100 décrit précédemment présente une forme coudée qui facilite l'introduction de la partie distale dans le forage de l'os maxillaire à l'intérieur de la bouche du patient. L'angle du coude formé entre les parties proximales et distales peut varier en fonction du site à atteindre. Le corps de l'insert selon l'invention peut en outre présenter d'autres formes adaptées à des accès de sites particuliers.

La forme de la portion annulaire formée au niveau de la partie distale de l'insert peut présenter différents profils. L'insert ultrasonique décrit précédemment comprend une portion annulaire à section croissante qui présente un profil incurvé (croissance de section de la portion annulaire non linéaire). La partie distale de l'insert selon l'invention peut également avoir une portion cylindrique qui s'élargit suivant un profil rectiligne (croissance linéaire de section de la portion cylindrique) ou autre.

## Revendications

1. Appareil de traitement dentaire à ultrasons comprenant au moins une pièce à main chirurgicale (200) reliée à un générateur d'ultrasons (300) comportant des moyens pour alimenter la pièce à main en liquide d'irrigation (305), ladite pièce à main comprenant des moyens pour générer des vibrations ultrasonores et des moyens pour distribuer le liquide d'irrigation délivré par le générateur d'ultrasons, ledit appareil de traitement dentaire comprenant en outre au moins un insert ultrasonique (100) destiné à soulever une membrane sinusienne par introduction dans un trou formé dans un os maxillaire, ledit insert comprenant un corps (101) s'étendant entre une partie proximale (102) adaptée pour être couplée mécaniquement avec une pièce à main chirurgicale (200) génératrice de vibrations ultrasonores et une partie distale (103) destinée à reproduire les vibrations ultrasonores transmises par la pièce à main,
la partie distale (103) comportant une portion annulaire (106) présentant une section qui s'agrandit vers l'extrémité libre de la partie distale (103) et dont l'extrémité est formée par une surface plane (107) sensiblement perpendiculaire à l'axe longitudinal (101b) de ladite partie distale et en ce que le corps (101) présente une courbure entre la partie proximale (102) et la parie distale (103), ledit insert (100) comprenant en outre un canal d'irrigation interne (105) débouchant sur ladite surface plane (107), la portion annulaire présentant une surface lisse, le canal d'irrigation interne (105) de l'insert étant en communication avec les moyens de distribution de liquide d'irrigation de ladite pièce à main et ledit insert étant couplé mécaniquement aux moyens de génération de vibrations ultrasonores de la pièce à main (200) de manière à mettre en cavitation le liquide d'irrigation délivré au niveau de la surface plane présente à l'extrémité de la portion annulaire de l'insert.

2. Appareil selon la revendication 1, dans lequel le corps de l'insert présente entre la partie proximale et la parie distale un angle de courbure d'au moins 45°.

3. Appareil selon la revendication 1 ou 2, dans lequel le canal d'irrigation interne (105) de l'insert débouche au centre de la surface plane (107).

4. Appareil selon l'une quelconque des revendications 1 à 3, dans lequel la surface plane (107) située à l'extrémité de la portion annulaire de la partie distale de l'insert présente à sa périphérie un bord arrondi (107a).

5. Appareil selon l'une quelconque des revendications 1 à 4, dans lequel la surface plane (107) située à l'extrémité de la portion annulaire de la partie distale de l'insert présente un diamètre de 3 mm environ.

## Patentansprüche

1. Ultraschallzahnbehandlungsgerät, umfassend mindestens ein chirurgisches Handstück (200), das an einen Ultraschallgenerator (300) angeschlossen ist, der Mittel zum Versorgen des Handstücks mit Spülflüssigkeit (305) aufweist, wobei das Handstück Mittel zum Erzeugen von Ultraschallvibrationen und Mittel zum Verteilen der Spülflüssigkeit, die von dem Ultraschallgenerator abgegebenen wird, aufweist, wobei das Zahnbehandlungsgerät ferner mindestens einen Ultraschalleinsatz (100) aufweist, der dazu bestimmt ist, eine Sinusmembran durch Einführen in ein Loch, das in einem Oberkieferknochen gebildet ist, anzuheben, wobei der Einsatz einen Körper (101) aufweist, der sich zwischen einem proximalen Teil (102), der geeignet ist, mechanisch mit einem chirurgischen Handstück (200), das Ultraschallvibrationen erzeugt, verbunden zu werden, und einem distalen Teil (103) erstreckt, der dazu bestimmt ist, die Ultraschallvibrationen zu reproduzieren, die von dem Handstück übertragen werden, wobei der distale Teil (103) einen ringförmigen Abschnitt (106) aufweist, der einen zunehmenden Querschnitt aufweist, der sich in Richtung des freien Endes des distalen Abschnitts (103) vergrößert und dessen Ende durch eine ebene Fläche (107) gebildet ist, die im Wesentlichen senkrecht zur Längsachse (101b) des distalen Abschnitts ist, und dadurch, dass der Körper (101) eine Krümmung zwischen dem proximalen Teil (102) und dem distalen Teil (103) aufweist, wobei der Einsatz (100) ferner einen inneren Spülkanal (105) aufweist, der auf der ebenen Fläche (107) mündet, wobei der ringförmige Abschnitt eine glatte Oberfläche aufweist, wobei der innere Spülkanal (105) des Einsatzes mit den Mitteln zum Verteilen der Spülflüssigkeit des Handstücks in Verbindung steht und der Einsatz mechanisch mit den Mitteln zum Erzeugen von Ultraschallvibrationen des Handstücks (200) derart verbunden ist, um die zugeführte Spülflüssigkeit an der ebenen Fläche, die am Ende des ringförmigen Abschnitts des Handstücks vorhanden ist, zu kavitieren.

2. Gerät gemäß Anspruch 1, wobei der Körper des Einsatzes zwischen dem proximalen Teil und dem distalen Teil einen Krümmungswinkel von mindestens 45° aufweist.

3. Gerät gemäß Anspruch 1 oder 2, wobei der innere Spülkanal (105) des Einsatzes in der Mitte der ebenen Fläche (107) mündet.

4. Vorrichtung gemäß einem der Ansprüche 1 bis 3, wobei die ebene Fläche (107), die sich am Ende des ringförmigen Abschnitts des distalen Teils des Einsatzes befindet, an ihrem Umfang eine abgerundete Kante (107a) aufweist.

5. Vorrichtung gemäß einem der Ansprüche 1 bis 4, wobei die ebene Fläche (107), die sich am Ende des ringförmigen Abschnitts des distalen Teils des Einsatzes befindet, einen Durchmesser von etwa 3 mm aufweist.

## Claims

1. Ultrasound dental treatment apparatus comprising at least one surgical handpiece (200) connected to an ultrasound generator (300) having means for supplying the handpiece with irrigation fluid (305), the handpiece comprising means for generating ultrasound vibrations and means for dispensing the irrigation liquid delivered by the ultrasound generator, said dental treatment apparatus further comprising at least one ultrasound insert (100) for lifting a sinus membrane by insertion into a hole in a maxillary bone, said insert comprising a body (101) extending between a proximal part (102) designed to be mechanically coupled to a surgical handpiece (200) which generates ultrasound vibrations, and a distal part (103) for reproducing the ultrasound vibrations transmitted by the handpiece, said distal part comprising an annular part (106) presenting a section which expands towards the free end of the distal part (103) and whose end is formed by a planar surface (107) that is substantially perpendicular to the longitudinal axis (101b) of the distal part, wherein the body (101) has a curvature between the proximal part (102) and the distal part (103), said insert (100) further comprising an internal irrigation channel (105) opening onto the planar surface (107) while the annular part presents a smooth surface, the internal irrigation channel (105) of the insert is in communication with the irrigation liquid dispensing means of the handpiece, and the insert is mechanically coupled to the ultrasound vibration generating means of the handpiece (200) in order to cavitate the irrigation liquid delivered to the planar surface present at the end of the annular part of the insert.

2. Apparatus according to claim 1, wherein the body of the insert has a bending angle of at least 45° between the proximal part and the distal part.

3. Apparatus according to claim 1 or 2, wherein the internal irrigation channel (105) of the insert opens at the center of the planar surface (107).

4. Apparatus according to any one of the claims 1 to 3, wherein the planar surface (107) at the end of the annular part of the distal part of the insert has a rounded edge (107a) at its periphery.

5. Apparatus according to any one of the claims 1 to 4, wherein the planar surface (107) at the end of the annular part of the distal part of the insert has a diameter of about 3 mm.
